# EUROPÄISCHE PATENTSCHRIFT

(11) **EP 1 425 143 B1**
(45) Veröffentlichungstag und Bekanntmachung des Hinweises auf die Patenterteilung: **16.03.2005**
(21) Anmeldenummer: 02764620.7
(22) Anmeldetag: 03.07.2002
(51) Int. Cl.: B26F 1/24, D04H 1/00

(54) **VLIESPERFORIERUNGSVORRICHTUNG NEBST VERFAHREN**
NON-WOVEN FABRIC PERFORATING DEVICE AND METHOD THEREFOR
DISPOSITIF DE PERFORATION DE NON-TISSE ET PROCEDE CORRESPONDANT

(30) Priorität: 03.07.2001 DE 20121445 U
(43) Veröffentlichungstag der Anmeldung: 09.06.2004
(73) Patentinhaber: Corovin GmbH, 31224 Peine (DE)
(72) Erfinder: MUTH, Mathias, 65197 Wiesbaden (DE); SODEMANN, Ralf, 31224 Peine (DE); ROY, Indra, CH-5445 Eggenwil (CH)
(86) Internationale Anmeldenummer: PCT/EP2002/007343
(87) Internationale Veröffentlichungsnummer: WO 2003/004229

(56) Entgegenhaltungen:
- EP-A- 1 048 419
- DE-A- 19 827 567
- DE-A- 19 856 223
- JP-A- 2 216 252
- US-A- 4 886 632

## Beschreibung

Die vorliegende Erfindung betrifft eine Vliesperforierungsvorrichtung mit einer Perforierungswalze und einer Gegenwalze sowie ein Verfahren zum Perforieren eines Vlieses, derartig hergestellte Vliese sowie Produkte, die derartige Vliese aufweisen.

Das Perforieren von Vliesen weist Vorteile auf, wenn es um Anwendungsgebiete wie Aufnahme von Flüssigkeiten bzw. Partikeln geht. Beispielsweise geht aus der US 5,858,504 ein Herstellungsverfahren, eine Herstellungsvorrichtung wie auch ein Vlies hervor, welches perforiert ist. Das Herstellungsverfahren sieht vor, dass mittels einer Nadelwalze und einer den Nadeln gegenüberliegenden Lochwalze ein Vlies perforiert wird. Dazu weist die Vorrichtung zwei S-Rollenanordnungen auf. Die S-Rollenanordnungen sind unterschiedlich schnell, so dass das durch beide S-Rollenanordnungen hindurch verlaufende Vlies aufgrund des Geschwindigkeitsunterschiedes verstreckt wird, bevor es perforiert wird. Nach dem Perforieren verbleibt das perforierte Vlies auf der Gegenwalze und wird zur zweiten S-Rollenanordnung geführt. Die Gegenwalze wie auch die Nadelwalze sind beide nicht beheizt, so dass das Vlies unterhalb der Schmelztemperatur des verwendeten Polymers perforiert wird.

Aufgabe der vorliegenden Erfindung ist es, eine Vliesperforierungsvorrichtung, ein Verfahren zum Perforieren eines Vlieses, ein Vlies sowie ein Produkt mit einem derartigen Vlies zur Verfügung zu stellen, welche für Anwendungen und Weiterverarbeitungen stabilisierte Perforierungen erzielt.

Diese Aufgabe wird mit einer Vliesperforierungsvorrichtung mit den Merkmalen des Anspruches 1, mit einem Verfahren zum Perforieren eines Vlieses mit den Merkmalen des Anspruches 8, mit einem Vlies mit den Merkmalen des Anspruches 10 gelöst. Weitere vorteilhafte Ausgestaltungen und Merkmale sind in den abhängigen Ansprüchen angegeben.

Eine erfindungsgemäße Vliesperforierungsvorrichtung weist eine Perforierungswalze und eine Gegenwalze auf. Die Perforierungswalze und die Gegenwalze bilden einen Spalt, durch den das zu perforierende Vlies hindurchgeführt und perforiert wird. Weiterhin weist die Vliesperforierungsvorrichtung eine Vlieszuführung und eine Vliesabführung auf. Die Vlieszuführung ist derart angeordnet, dass ein zu perforierendes Vlies zuerst auf die Gegenwalze geführt wird, bevor es in den Spalt geführt wird. Die Vliesabführung ist derart angeordnet, dass das perforierte Vlies nach Verlassen des Spaltes auf der Perforierungswalze vorzugsweise für einen gewissen Zeitraum liegen bleibt.

Vorzugsweise ist die Perforierungswalze eine Nadelwalze. Die Nadeln weisen insbesondere einen konzentrischen Durchmesser auf. Daneben können die Nadeln auch andere Durchmesser-Geometrien aufweisen, beispielsweise sternförmig, rechteckförmig oder Halbwinkel sein. Gemäß einer Weiterbildung wird vorgesehen, dass ein zu perforierendes Vlies auf der Gegenwalze einen Umschlingungswinkel von mindestens 45 Grad hat, vorzugsweise einen Umschlingungswinkel größer 90 Grad. Weiterhin ist es bevorzugt, dass ein perforiertes Vlies auf der Perforierungswalze einen Umschlingungswinkel von mindestens 45 Grad hat, vorzugsweise einen Umschlingungswinkel größer 90 Grad.

Eine Weiterbildung sieht vor, dass die Vliesperforierungsvorrichtung eine Beheizung für das zu perforierende Vlies hat.

Eine andere Weiterbildung sieht vor, dass zwischen der Vlieszuführung und der Gegenwalze eine Zuspannung definiert einstellbar ist, die auf das zu perforierende Vlies wirkt. Eine zusätzliche Ausgestaltung sieht vor, dass die Vliesperforierungsvorrichtung zwischen der Perforierungswalze und der Vliesabführung eine Zugspannung definiert einstellbar aufweist, die auf das perforierte Vlies wirkt. Vorzugsweise ist eine Zugspannung zwischen der Vlieszuführung und der Gegenwalze größer eingestellt gegenüber einer Zugspannung, die zwischen der Perforierungswalze und der Vliesabführung herrscht.

Ein hiervon insbesondere unabhängiger Gedanke sieht vor, dass ein Verfahren zum Perforieren eines Vlieses zur Verfügung gestellt wird, wobei das Vlies von einer Vlieszuführung in einen Spalt zwischen einer Perforierungswalze und einer Gegenwalze geführt wird, das Vlies in dem Spalt perforiert wird, das Vlies nach dem Perforieren zu einer Vliesabführung zugeführt wird und das Vlies vor dem Perforieren die Gegenwalze mit einem Umschlingungswinkel größer 45 Grad umschlingt. Das Vlies wird zwischen der Vlieszuführung und der Gegenwalze zwischen 1,5 % und 10 % vorgedehnt, bevor es die Gegenwalze umschlingt. Die Vordehnung erfolgt insbesondere in Maschinenrichtung (MD), kann aber auch in Querrichtung (CD) erfolgen.

Ein vorverdehntes Vlies, das auf die Gegenwalze geführt wird, hat den Vorteil, dass aufgrund der zwischen der Gegenwalze und dem Vlies herrschenden Reibkraft das vorgedehnte Vlies fixiert wird. Die Vorverdehnung ist insbesondere daher vorteilhaft, da verdrängte Fasern durch die anschließende Fixierung in ihrer Position verbleiben. Diese Fixierung wird aufrecht erhalten zumindest bis die Perforierungswalze mit der Gegenwalze das Vlies perforiert. Beispielsweise wird als Vlieszuführung eine S-Walzenanordnung genutzt. Über die S-Walzenanordnung wird eine Zugspannung zwischen dieser Vlieszuführung und der Gegenwalze auf das Vlies aufgebracht. Durch die zwischen dem Vlies und der Gegenwalze herrschenden Reibkräfte verbleibt das Vlies in seiner Position. Diese Art der Fixierung erlaubt es insbesondere, dass die zwischen der Vlieszuführung und der Gegenwalze herrschende, auf das Vlies wirkende Zugkraft unabhängig einstellbar ist von nach der Perforierungswalze und Gegenwalze angeordneten weiteren Vorrichtungen wie Umlenkungsrollen etc.

Vorzugsweise umschlingt das Vlies nach der Perforierung die Perforierungswalze mit einem Umschlingungswinkel von mindestens 45 Grad. Dazu wird das Vlies, das vorher auf der Gegenwalze auflag, während des Perforierens bzw. kurz zuvor oder danach auf die Perforierungswalze umgelenkt. Die über einen gewissen Umdrehungsweg der Perforierungswalze fortgesetzte Fixierung des Vlieses auf der Perforierungswalze führt zu einer weiteren Stabilisierung der Perforationen im Vlies selbst. Vorzugsweise umschlingt das Vlies die Perforierungswalze mit einem Umschlingungswinkel zwischen 90 Grad und 270 Grad.

Gemäß einem weiteren Gedanken der Erfindung wird ein Vlies mit der Vliesperforierungsvorrichtung hergestellt bzw. mit einem Verfahren, wie oben beschrieben, hergestellt. Das Vlies weist Perforationen auf, die annähernd kreisrund sind. Insbesondere weisen diese Perforationen ein Achsenverhältnis MD zu CD auf, das annähernd 1 ist, vorzugsweise zwischen 1 und 1,18 liegt. Vorzugsweise wird ein derartiges Vlies in einem Produkt eingesetzt, wobei das Vlies stabilisierte trichterförmige Öffnungen an einer Produktoberfläche aufweist. Diese Öffnungen sind durch die Perforationen während des Perforationsverfahrens bzw. in der Vliesperforierungsvorrichtung entstanden.

insbesondere ist ein derartiges perforiertes Vlies einsetzbar in Produkten, die im Hygienebereich Anwendung finden. Dieses können Windeln, Binden, Inkontinenzartikel oder anderes sein. Auch bei Medikalartikeln wie Abdeckungen, Schutzbekleidungen, Teilen davon sowie im Haushaltsbereich, beispielsweise bei Wischtüchern, findet ein derartiges Vlies als Bestandteil eines Produktes Verwendung. Weiterhin ist es überall insbesondere dort einsetzbar, wo einerseits definierte Lochgrößen bevorzugt werden, andererseits auch Eigenschaften wie Flüssigkeitsaufnahme bzw. Partikelaufnahme von Bedeutung sind. Beispielsweise kann die Vliesoberfläche hydrophil ausgerüstet sein, beispielsweise mittels einer Additivierung oder eines Auftrages. Auch kann das verwendete Vlies elektrostatisch aufgeladen werden. Weiterhin kann das Vlies ein- oder mehrlagig sein, ein Laminat mit einem Film oder anderen Trägermaterialien bilden. Insbesondere kann ein derartiges Material auch dort eingesetzt werden, wo nur bestimmte Bereiche flüssigkeits- bzw. dampfdurchlässig sein sollen.

Beispiele von vorteilhaft zu perforierenden Materialien für zumindest zweilagige Materialien gehen aus der nachfolgenden Tabelle hervor:

| Material der zweiten Lage | Material der ersten Lage |
|---|---|
| Spinnvlies PP . | Spinnvlies PE |
| Cardiertes PP | Spinnvlies PE |
| Spinnvlies PP | Spinnvlies BICO, z.B. PP/PE |
| Spinnvlies PP | Kardiertes BICO z.B. PP/PE vorzugsweise mit PET (z.B. zwischen 10% bis 40%) |
| Film PP | Film PE |
| Vliesstoff PP | Film PE |
| Spinnvlies BICO PP/PE | Spinnvlies PE |
| Spinnvlies BICO PP/PE | Film PE |
| Vliesstoff PP | Hochvoluminöses Vlies BICO PP/PE |
| Spinnvlies HDPE | Kardiertes BICO, z.B. PP/PE, vorzugsweise mit PET (z. B. zwischen 10% bis 40%) |
| Vliesstoff PP | Kardiertes PE |
| Vliesstoff PP | PP Vliesstoff mit niedrigem Schmelzpunkt, z.B. Softspun™ |
| Spinnvlies BICO PP/PE | Kardiertes BICO PP/PE |

Getestet wurden Flächengewichte wie folgt:

| Flächengewicht der zweiten Lage [gsm] | Flächengewicht der ersten Lage [gsm] |
|---|---|
| Von etwa 10 bis etwa 50 | Von etwa 10 bis etwa 50 |

Vorzugsweise hat die erste Lage ein Flächengewicht, das höher ist als das Flächengewicht der zweiten Lage.

Versuchergebnisse waren wie folgt:

| Muster | Flächengewicht [g/m²] | Dehnungsrate [%] | Achsenverhältnis MD/CD | Lochfläche [mm²] | Offene Fläche [%] |
|---|---|---|---|---|---|
| A | 43 | 2,8 | 1,01 | 1,45 | 22,3 |
| B | 43 | 1,7 | 1,13 | 1,46 | 22,4 |
| C | 30 | 2,2 | 1,04 | 1,38 | 21,1 |
| D | 25 | 5,6 | 1,11 | 1,31 | 20, 7 |
| E | 30 | 3, 5 | 1,16 | 1,24 | 18,9 |

Wobei:
- Dehnungsrate:: Verhältnis der Warenbreite unmittelbar vor dem Spalt zur Originalwarenbreite in %
- Achsenverhältnis:: Durchschnittliches Verhältnis der Längs- zur Querachse der Löcher bei Annahme einer Ellipse mit MD = Maschinenrichtung und CD = Querrichtung
- Lochfläche:: Durchschnittliche Fläche der Löcher in mm² (berechnet mit Bildbearbeitungsprogramm Image-Pro Plus Version 4.5 der Fa. MediaCybernetics)
- Offene Fläche:: Durchschnittliches Verhältnis der Lochfläche zur Gesamtfläche des Vlieses in %

Die Eintauchtiefe der Nadeln in die gelochte Gegenwalze betrug bei allen Versuchen 2,7 Millimeter. Die verwendeten Muster waren Vorlagenvliese, die jeweils als Spinnvliese hergestellt wurden.
- Muster A:: zweilagiges nach dem Docan-Prozeß hergestelltes Material, wobei eine obere Lage 20 gsm Vlies aus PP und eine untere Lage 23 gsm Vlies aus PP/PE-Bico aufweist;
- Muster B:: wie Muster A;
- Muster C:: einlagiges 30 gsm Vlies aus PP, hergestellt nach dem Docan-Prozeß;
- Muster D:: wie C aber nur 25 gsm;
- Muster E:: einlagiges Vlies mit 30 gsm aus PP, hergestellt nach dem S-Tex-Verfahren.

Wie insbesondere das Verhältnis der Lochgröße MD/CD zeigt, gelingt es auch, besonders runde Öffnungen der Perforierungen zu stabilisieren. Die Lochdurchmesser betragen in MD zwischen 1 und 1,8 mm und in CD zwischen 0,8 und 1,7 mm. Vorzugsweise weist das Vlies eine Vorspannung auf, die für eine Dehnungsrate zwischen 2% und 3% sorgt.

Einen weiteren Einfluß auf die Lochgrößen hat die Geschwindigkeit, mit der das Vlies durch die Vliesperforierungsvorrichtung hindurchläuft. Das Vlies wurde mit Geschwindigkeiten zwischen 5 m/s bis zu 130 m/s hindurchgeführt. Als vorteilhaft haben sich Geschwindigkeiten zwischen 45 m/s und 120 m/s, insbesondere zwischen 60 m/s und 95 m/s zur Herstellung einer stabilen Perforierung herausgestellt. Bei Lochdurchmessern, die sich in einem Bereich von unter 0,5 mm bewegen, ist eine höhere Betriebsgeschwindigkeit einstellbar. Hier sind Geschwindigkeiten bis 200 m/s einstellbar, vorzugsweise Geschwindigkeiten über 150 m/s. Die Lochdurchmesser sind beispielsweise dann in einem Bereich zwischen 0,5 und 0,1 mm. Die Gegenwalze weist vorzugsweise eine Temperatur zwischen 45°C und 95°C, insbesondere zwischen 55°C und 75°C hat.

Gemäß einem weiteren Gedanken weist eine Vliesperforierungsvorrichtung eine Zuführung für ein zu perforierendes Vlies auf, die so angeordnet ist, dass das Vlies über einen Umschlingungswinkel von über 120°, vorzugsweise über 150° entlang der Gegenwalze geführt wird, bevor eine Perforierung durchführbar ist. Dadurch gelingt es insbesondere, dass bei einer erwärmten Gegenwalze das Vlies angewärmt der Perforierungswalze zugeführt wird. Darüber hinaus wird aufgrund der Umschlingung eine Spannung im Material, das sich in Kontakt mit der Gegenwalze befindet, abgesenkt. Dadurch gelingt eine besonders stabile Perforierung.

Gemäß einer weiteren Ausbildung weist die Gegenwalze eine Beschichtung auf, vorzugsweise eine Gummierung. Die Beschichtung ist insbesondere zwischen 1,5 mm und 15 mm dick, insbesondere mindestens 4 mm. Die Erhebungen der Perforierungswalze können in die Beschichtung eingreifen. Vorzugsweise greifen diese in eine Tiefe von etwa 2,5 bis etwa 6 mm ein.

Ein zweilagiges zu perforierendes Laminat wird gemäß einer Ausgestaltung in einem integrierten Herstellungsverfahren produziert. Beispielsweise wird bei einer Vliesherstellung eine Spinnvliesmaschine mit einem oder mehreren Balken zur Verfügung gestellt. Mittels eines der Balken wird beispielsweise eine Polymer-Mischung mit niedrigem Schmelzpunkt und mittels eines zweiten Balkens ein BICO PP/PE-Vlies hergestellt. Weiterhin kann auch auf ein vorgefertigtes Material eine zweite Lage aufgebracht und dann anschließend perforiert werden. Weiterhin besteht die Möglichkeit, die erste und die zweite Lage inline herzustellen und in einem davon getrennten Arbeitsvorgang zu perforieren. Wie am Beispiel von Vliesstoff dargestellt, besteht weiterhin die Möglichkeit, Kombinationen von Film und Vliesstoff zu verwenden. Beispielsweise kann ein Film auf ein beispielsweise kardiertes Vlies extrudiert werden und anschließend einer Perforierungseinheit zugeführt werden.

Gemäß einer weiteren Ausgestaltung sind die Fasern der ersten Lage zumindest teilweise mit Fasern eines Vlieses der zweiten Lage vermengt, insbesondere in Form eines Ineinandergreifens. Während beispielsweise zwei getrennt voneinander hergestellte Vlieslagen zwischen sich eine Materialgrenze aufweisen können, weisen die beiden zum Teil miteinander vermengten Vlieslagen einen Materialübergang auf. Außerhalb des Materialüberganges weisen die eine wie die andere Lage jeweils nur ein thermoplastisches Material auf. Ein derartiger Aufbau wird insbesondere mittels eines Inlineverfahrens hergestellt. Vorzugsweise weist die perforierte Struktur einen Phasenübergang oder gemäß einer weiteren Ausgestaltung eine beispielsweise vollständige Vermischung der Fasern zumindest teilweise im Bereich der Perforierung auf. Vorzugsweise werden die erste und die zweite Lage auf gleiche Weise hergestellt. Beide Lagen sind beispielsweise extrudierte Vliese, die auf der gleichen Maschine hergestellt werden. Auch besteht die Möglichkeit, unterschiedliche Materialien mit jeweils unterschiedlichen Eigenschaften zu einer perforierten Struktur bilden zu können. Während das eine Vlies PP zumindest zum überwiegenden Teil aufweist, ist das andere Vlies zum überwiegenden Teil aus HDPE oder DAPP bestehend. Darüber hinaus bestehen Kombinationsmöglichkeiten von verschiedenen Herstellungsweisen von Vliesen, insbesondere Verwendung von hochvoluminösen Stapelfaservliesen mit Spinnvliesen oder auch schmelzgeblasenes Vlies mit Spinnvlies sowie weitere Kombinationen.

Beispiele für eine Anwendung des Laminats bzw. des Vlieses in einem Produkt sind Hygieneartikel, Sanitär- und Haushaltsartikel, insbesondere Wischtücher, medizinische Produkte, Oberflächenanwendungen bei Produkten, Filtermaterialien, Schutzbekleidungen, Geotextilien, Wegwerfprodukte.

Weitere vorteilhafte Ausgestaltungen und Weiterbildungen gehen aus der nachfolgenden Zeichnung hervor, die jedoch die Erfindung in ihrer Ausgestaltung nicht beschränken sollen. Die dort dargestellten Merkmale und Weiterbildungen sind auch mit den weiter oben beschriebenen zu ansonsten nicht näher ausgeführten Ausgestaltungen der Erfindung kombinierbar.

Es zeigen:
- Figur 1: eine Vliesperforierungsvorrichtung mit einem Abwickler und einem Aufwickler,
- Figur 2: einen Ausschnitt aus Figur 1, aus dem ein Wechsel der Lage des zu perforierenden Vlieses bzw. perforierten Vlieses von einer Gegenwalze zu einer Perforierungswalze der Vliesperforierungsvorrichtung hervorgeht,
- Figur 3a, b: ein zu perforierendes einlagiges Material und
- Figur 4a, b: ein zu perforierendes zweilagiges Material.

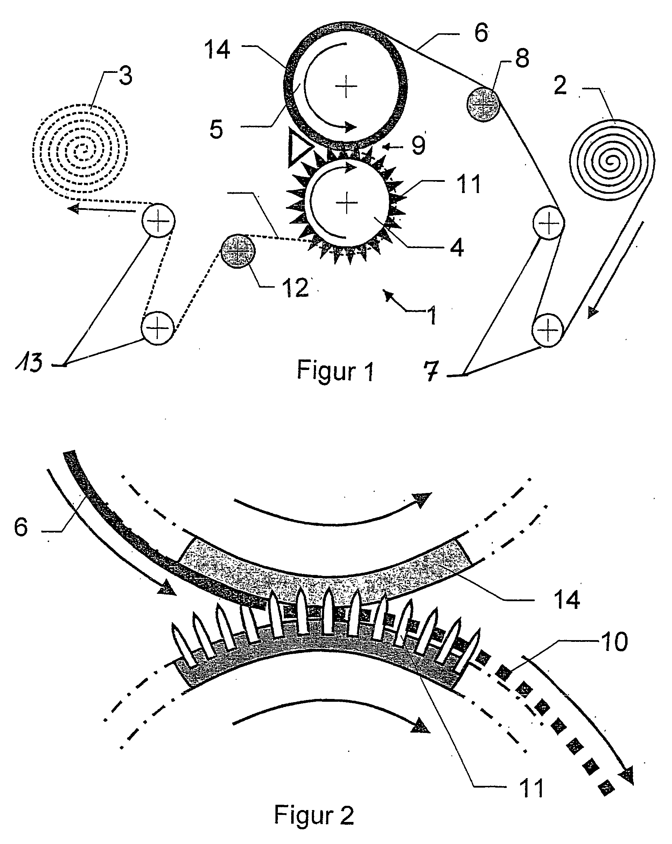

Fig. 1 zeigt eine Vliesperforierungsvorrichtung 1 mit einem Abwickler 2 und einem Aufwickler 3. Anstatt eines Abwicklers 2 bzw. eines Aufwicklers 3 können auch andere Fertigungsstationen vorgesehen sein. Zum Beispiel kann eine direkte Spinnvlies-, Stapelfaservlies- und/oder Meltblownvliesfertigung der Vliesperforierungsvorrichtung 1 vorgelagert sein. Anstatt des Aufwicklers 3 kann auch eine Konfektionierungsmaschine, eine Festooning-Einheit, eine Sprüheinheit, eine Produziervorrichtung für ein Produkt wie ein Wischtuch oder anderes oder anderes vorgesehen sein. Vom Abwickler 2 wird ein vorzugsweise vorverfestigtes Vlies oder Laminat aus ein oder mehreren Vliesen bzw. Vliesen mit einem anderen Material, beispielsweise einem Film, zu einer Perforierungswalze 4 zugeführt. Die Perforierungswalze 4 weist Perforierungseinrichtungen auf. In diesem Falle ist die Perforierungswalze 4 als Nadelwalze ausgestaltet. Der Perforierungswalze 4 gegenüberliegend ist eine Gegenwalze 5 angeordnet. Gegenwalze 5 wie Perforierungswalze 4 weisen vorzugsweise die gleiche Umdrehungsgeschwindigkeit auf. Das vom Abwickler 2 zugeführte zu perforierende Vlies 6 wird über Zuführungsrollen 7 auf die Gegenwalze 5 aufgebracht. Die Zuführungsrollen 7 sind insbesondere derartig ausgestaltet, dass zwischen einer Vlieszuführung 8 und der Gegenwalze 5 eine definierbare Zugspannung auf das zu perforierende Vlies 6 einstellbar ist. Vorzugsweise weist die Vlieszuführung 8 eine Zugmesswalze auf. Das zu perforierende Vlies 6 wird von der Vlieszuführung 8 kommend so auf die Gegenwalze 5 aufgebracht, dass es letztere zumindest teilweise umschlingt. Vorzugsweise ist die Gegenwalze 5 direkt aufgeheizt. Die Temperatur der Gegenwalze 5 ist insbesondere so, dass sie unterhalb der Erweichungs- bzw. Schmelztemperatur des verwendeten Polymers des zu perforierenden Vlieses 6 bzw. verwendeten Laminates liegt. Vorzugsweise wird eine derartige Temperierung während des Mitführens des zu perforierenden Vlieses 6 durch die Gegenwalze 5 übertragen. Weiterhin besteht die Möglichkeit, dass durch nicht näher dargestellte Beheizungseinrichtungen das Vlies vor dem Perforieren aufgeheizt wird. Dabei ist angestrebt, dass das Vlies eine Temperatur annimmt, die unterhalb der Schmelztemperatur des verwendeten Polymers liegt. Von der Gegenwalze 5 wird das zu perforierende Vlies 6 in einen Spalt 9 geführt, der durch die Perforierungswalze 4 und die Gegenwalze 5 gebildet wird. In dem Spalt 9 wird das zu perforierende Vlies 6 perforiert und gemäß dieser Vliesperforierungsvorrichtung 1 auf die Perforierungswalze 4 hinübergeführt. Von der Perforierungswalze 4 wird das perforierte Vlies 10 von Nadeln 11 der Perforierungswalze 4 zu einer Vliesabführung 12 geführt. Der Vliesabführung 12 sind so wie dargestellt weitere Abführungsrollen 13 nachgeordnet. Vorzugsweise weist die Vliesabführung 12 eine Zugmesswalze auf. Von den Abführungsrollen 13 gelangt das perforierte Vlies 10 zu dem Aufwickler 3. Vorzugsweise herrscht auf das perforierte Vlies 10 eine Zugspannung, die kleiner ist als die Zugspannung, die auf das zu perforierende Vlies 6 wirkt. Das perforierte Vlies 10 wird auf der Perforierungswalze 4 entlang eines gewissen Umschlingungswinkels mitgeführt. Auf diese Weise stabilisiert sich die Perforierung im Vlies. Durch eine entsprechende Aufbringung einer Zugkraft über die Vliesabführung 12 auf das zwischen der Vliesabführung 12 und der Perforierungswalze 4 angeordnete perforierte Vlies 10 werden die Perforationen vorzugsweise zusätzlich stabilisiert. Weiterhin kann die Perforierungswalze 4 ebenfalls temperiert sein. Die Wärmezufuhr an das perforierte Vlies 10 bzw. das entsprechende Laminat stabilisiert die auf diese Weise verdrängten Vliesfasern.

Gemäß einer Weiterbildung ist die Temperatur der Gegenwalze 5 mindestens 40 Grad Celsius höher als die der Perforierungswalze 4. Eine weitere Ausgestaltung sieht vor, dass die Gegenwalze 5 temperiert ist, die Perforierungswalze 4 jedoch nicht. Vielmehr kann letztere auch gekühlt sein, beispielsweise auf eine Temperatur von etwa 18 Grad Celsius und niedriger.

Fig. 2 zeigt einen Ausschnitt aus Fig. 1, in dem die Perforierungswalze 4, die Gegenwalze 5 sowie das zu perforierende Vlies 6, das das perforierte Vlies 10 wird, näher dargestellt sind. Teile der Perforierungswalze 4 greifen in die Gegenwalze 5 ein. Durch diesen Eingriff wird das Vlies perforiert, das in den zwischen der Gegenwalze 5 und der Perforierungswalze 4 gebildeten Spalt eingeführt wird. Die Perforation erfolgt vorzugsweise durch Verdrängung der Vliesfasern. Dadurch bleibt die Faserstruktur zumindest an der Oberfläche des Vlieses erhalten. Insbesondere werden die Fasern nur verdrängt, ohne dass sie angeschmolzen oder erweicht werden. Die Eigenschaften der Fasern wie beispielsweise eine bevorzugte Flüssigkeitsabfuhr entlang einer Faser bleiben dadurch bestehen. Durch das Umführen des Vlieses von der Gegenwalze 5 auf die Perforierungswalze 4 werden die Perforationen als solches in einem stabilisierten Zustand gehalten. In diesem Falle greifen Nadeln 11 in das Vlies ein. Der Eingriff der Nadeln 11 erfolgt vorzugsweise auch in eine Beschichtung 14 der Gegenwalze 5. Auch kann die Beschichtung 14 selbst Löcher aufweisen, die den Nadeln 11 gegenüberliegend angeordnet sind.

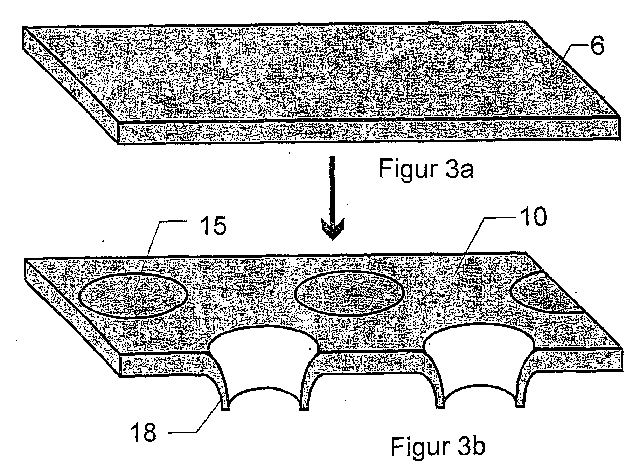

Fig. 3a und 3b zeigt ein einlagiges Vlies. Fig. 3a zeigt das zu perforierende Vlies 6, während Figur 3 b das perforierte Vlies 10 darstellt. Aus Fig. 3b ist zu erkennen, dass Perforationen 15 kegelförmig sein können. Die Art der Perforation stabilisiert diese kegelförmigen Gebilde 18, so dass diese sich als Erhebung vom Vliesgrund abheben.

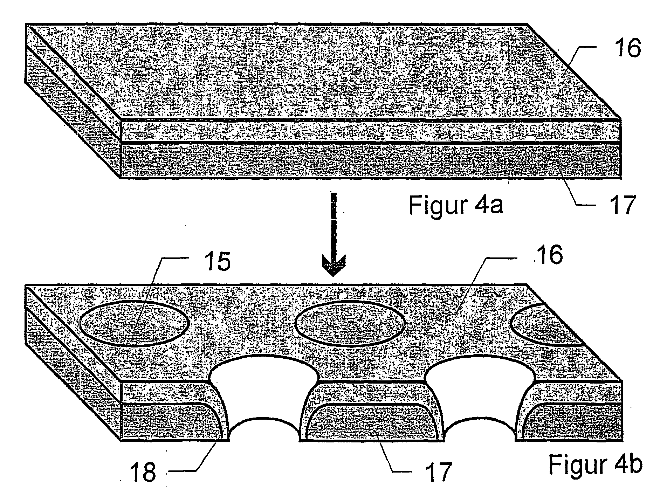

Fig. 4a und 4b zeigen eine Perforierung eines zweilagigen Materials, wobei eine erste Lage 16 ein Vlies ist und eine zweite Lage 17 ein Vlies oder ein anderes Material sein kann. Beispielsweise kann die zweite Lage ein Film sein. Auch können die erste Lage 16 und die zweite Lage 17 unterschiedliche oder gleiche Vliesarten sein. Die erste Lage 15 weist Perforationen 15 auf, die sich als kegelförmige Gebilde 18 in die zweite Lage 17 erstrecken. Die Gebilde 18 sind vorzugsweise so ausgeführt, dass sie nicht aus der Oberfläche der zweiten Lage 17 herausragen. Vorzugsweise bildet die zweite Lage 17 annähernd vollständig die Oberfläche ohne Material der ersten Lage 15. Gemäß einer Weiterbildung kann das zweilagige Material von einem trichterförmigen Gebilde 18 zum nächsten trichterförmigen Gebilde 18 eine leichte Wellung auf zumindest derjenigen Seite aufweisen, zu der das trichterförmige Gebilde 18 sich erhebt. Die gegenüberliegende Seite ist dagegen vorzugsweise annähernd eben.

## Patentansprüche

1. Vliesperforierungsvorrichtung (1) mit einer Perforierungswalze (4) und einer Gegenwalze (5), wobei die Perforierungswalze (4) und die Gegenwalze (5) einen Spalt (9) bilden, durch den ein zu perforierende Vlies (6) hindurchgeführt und perforiert wird, und wobei die Vliesperforierungsvorrichtung (1) eine Vlieszuführung (8) und eine Vliesabführung (12) aufweist,
**dadurch gekennzeichnet, dass**
die Vlieszuführung (8) derart angeordnet ist, dass das zu perforierende Vlies (6) zuerst auf die Gegenwalze (5) geführt wird, bevor es in den Spalt (9) geführt wird, und die Vliesabführung (12) derart angeordnet ist, dass das perforierte Vlies (10) nach Verlassen des Spaltes (9) auf der Perforierungswalze (4) liegen bleibt.

2. Vliesperforierungsvorrichtung (1) nach Anspruch 1, **dadurch gekennzeichnet, dass** die Perforierungswalze (4) eine Nadelwalze ist.

3. Vliesperforierungsvorrichtung (1) nach Anspruch 1 oder 2, **dadurch gekennzeichnet, dass** das zu perforierende Vlies (6) auf der Gegenwalze (5) einen Umschlingungswinkel von mindestens 45° hat, vorzugsweise einen Umschlingungswinkel größer 90°.

4. Vliesperforierungsvorrichtung (1) nach einem der Ansprüche 1, 2 oder 3, **dadurch gekennzeichnet, dass** das perforierte Vlies (10) auf der Perforierungswalze (4) einen Umschlingungswinkel von mindestens 45° hat, vorzugsweise einen Umschlingungswinkel größer 90°.

5. Vliesperforierungsvorrichtung (1) nach einem der vorhergehenden Ansprüche, **dadurch gekennzeichnet, dass** die Vliesperforierungsvorrichtung (1) eine Beheizung für das zu perforierende Vlies (6) hat.

6. Vliesperforierungsvorrichtung (1) nach einem der vorhergehenden Ansprüche, **dadurch gekennzeichnet, dass** zwischen der Vlieszuführung (8) und der Gegenwalze (5) eine Zugspannung, die auf dazu perforierende Vlies (6) wirkt, definiert einstellbar ist.

7. Vliesperforierungsvorrichtung (1) nach einem der vorhergehenden Ansprüche, **dadurch gekennzeichnet, dass** zwischen der Perforierungswalze (4) und der Vliesabführung (12) eine Zugspannung, die auf das perforierte Vlies (10) wirkt, definiert einstellbar ist.

8. Verfahren zum Perforieren eines Vlieses (6), wobei
- das Vlies (6) von einer Vlieszuführung (8) in einen Spalt (9) zwischen einer Perforierungswalze (4) und einer Gegenwalze (5) geführt wird,
- das Vlies in dem Spalt (9) perforiert wird,
- das Vlies nach dem Perforieren zu einer Vliesabführung (12) zugeführt wird und
- das Vlies vor dem Perforieren die Gegenwalze (5) mit einem Umschlingungswinkel größer 45° umschlingt und
- das Vlies (6) zwischen der Vlieszuführung (8) und der Gegenwalze (5) zwischen 1,5% und 10% vorgedehnt wird, bevor es die Gegenwalze (5) umschlingt,
**dadurch gekennzeichnet, dass** das Vlies nach der Perforierung die Perforierungswalze (4) mit einem Umschlingungswinkel von mindestens 45° umschlingt.

9. Verfahren nach Anspruch 8, **dadurch gekennzeichnet, dass** das Vlies die Perforierungswalze (4) mit einem Umschlingungswinkel zwischen 90° und 270° umschlingt.

10. Vlies (10) hergestellt mit einer Vliesperforierungsvorrichtung (1) nach Anspruch 1 und/oder mit einem Verfahren nach Anspruch 8, **dadurch gekennzeichnet, dass** das Vlies (10) eine Dehnungsrate von 1,7% bis 5,6%, vorzugsweise von 2% - 3% aufweist und dass die Perforationen ein Achsenverhältnis MD/CD zwischen 1 und 1,18 haben.

## Claims

1. Nonwoven perforating device (1) with a perforating roller (4) and a counter roller (5), whereby the perforating roller (4) and the counter roller (5) generate a gap (9), through which a nonwoven (6) to be perforated is fed and perforated, and whereby the nonwoven perforating device (1) comprises a nonwoven feeding (8) and a nonwoven lead-away guiding (12),
**characterised in that**
the nonwoven feeding (8) is arranged such that the nonwoven (6) to be perforated is fed firstly onto the counter roller (5) before it is fed into the gap (9) and the nonwoven lead-away guiding (12) is arranged such that the perforated nonwoven (10) rests on the perforating roller (4) after leaving the gap.

2. Nonwoven perforating device (1) according to claim 1, **characterised in that** the perforating roller (4) is a needle roller.

3. Nonwoven perforating device (1) according to claim 1 or claim 2, **characterised in that** the nonwoven (6) to be perforated has a contact arc of at least 45° on the counter roller (5), preferably a contact arc larger than 90°.

4. Nonwoven perforating device (1) according to claim 1, 2 or 3, **characterised in that** the perforated nonwoven (10) has a contact arc of at least 45° on the perforating roller (4), preferably a contact arc larger than 90°.

5. Nonwoven perforating device (1) according to one of the preceding claims, **characterised in that** the nonwoven perforating device (1) has a heating for the nonwoven (6) to be perforated.

6. Nonwoven perforating device (1) according to one of the preceding claims, **characterised in that** a tensile strength between the nonwoven feeding (8) and the counter roller (5), which affects the nonwoven(6) to be perforated, is adjustable in a defined manner.

7. Nonwoven perforating device (1) according to one of the preceding claims, **characterised in that** a tensile strength between the perforating roller (4) and the nonwoven lead-away guiding (12), which affects the perforated nonwoven (10), is adjustable in a defined manner.

8. Method for perforating a nonwoven (6), whereby
- the nonwoven (6) is fed from a nonwoven feeding (8) into a gap (9) between a perforating roller (4) and a counter roller (5),
- the nonwoven is perforated in the gap (9),
- the nonwoven is fed to a nonwoven lead-away guiding (12) after being perforated and
- the nonwoven wraps the counter roller (5) with a contact arc larger than 45° before being perforated and
- the nonwoven (6) is pre-stretched between 1.5 % and 10 % between the nonwoven feeding (8) and the counter roller (5), before it wraps the counter roller (5),
**characterised in that**
the nonwoven wraps the perforating roller (4) with a contact arc of at least 45° after being perforated.

9. Method according to claim 8, **characterised in that** the nonwoven wraps the perforating roller (4) with a contact arc between 90° and 270°.

10. Nonwoven (10) manufactured with a nonwoven perforating device (1) according to claim 1 and/or with a method according to claim 8, **characterised in that** the nonwoven (10) comprises an elongation rate of 1.7 % to 5.6 %, preferably of 2 % to 3 % and that the perforations have an axis ratio MD/CD between 1 and 1.18.

## Revendications

1. Dispositif de perforation de non-tissé (1) comportant un cylindre de perforation (4) et un contre-cylindre (5), le cylindre de perforation (4) et le contre-cylindre (5) forment une fente (9) au travers de laquelle un non-tissé à perforer (6) est guidé et perforé, et le dispositif de perforation de non-tissé (1) présente une alimentation de non-tissé (8) et une évacuation de non-tissé (12),
**caractérisé en ce que**
l'alimentation de non-tissé (8) est disposée de manière à ce que le non-tissé à perforer (6) est d'abord guidé sur le contre-cylindre (5) avant d'être guidé sur la fente (9) et l'évacuation de non-tissé (12) est disposée de manière à ce que le non-tissé perforé (10) reste sur le cylindre de perforation (4) après avoir quitté la fente (9).

2. Dispositif de perforation de non-tissé (1) selon la revendication 1 **caractérisé en ce que** le cylindre de perforation (4) est un rouleau à aiguilles.

3. Dispositif de perforation de non-tissé (1) selon la revendication 1 ou 2 **caractérisé en ce que** le non-tissé à perforer (6) a sur le contre-cylindre (5) un angle d'enroulement d'au moins 45°, préférentiellement plus grand que 90°.

4. Dispositif de perforation de non-tissé (1) selon l'une des revendications 1, 2 ou 3 **caractérisé en ce que** le non-tissé perforé (10) a sur le cylindre de perforation un angle d'enroulement d'au moins 45°, préférentiellement plus grand que 90°.

5. Dispositif de perforation de non-tissé (1) selon l'une des revendications précédentes **caractérisé en ce que** le dispositif de perforation de non-tissé (1) a un chauffage pour le non-tissé à perforer (6).

6. Dispositif de perforation de non-tissé (1) selon l'une des revendications précédentes **caractérisé en ce que** la tension qui agit sur le non-tissé à perforer (6) situé entre l'alimentation de non-tissé (8) et le contre-cylindre (5) est ajustable de manière définie.

7. Dispositif de perforation de non-tissé (1) selon l'une des revendications précédentes **caractérisé en ce que** la tension qui agit sur le non-tissé perforé (10) situé entre le cylindre de perforation (4) et l'évacuation de non-tissé (12) est ajustable de manière définie.

8. Procédé de perforation de non-tissé (6), où
- le non-tissé (6) est guidé par une alimentation de non-tissé (8) sur la fente (9) entre un cylindre de perforation (4) et un contre-cylindre (5),
- le non-tissé est perforé sur la fente (9),
- le non-tissé est conduit après la perforation à l'évacuation de non-tissé (12) et où
- le non-tissu s'enroule autour du cylindre de perforation (4) avec un angle d'enroulement d'au moins 45° après la perforation et
- le non-tissé (6) est pré-élargit entre 1,5% et 10% entre l'alimentation de non-tissé (8) et le contre-cylindre (5) avant qu'il s'entoure autour du contre-cylindre (5).

9. Procédé selon la revendication 8 **caractérisé en ce que** le non-tissé s'enroule autour du cylindre de perforation (4) avec un angle d'enroulement compris entre 90° et 270°.

10. Non-tissé (10) produit à l'aide d'un dispositif de perforation (1) selon la revendication 1 et/ou à l'aide d'un procédé selon la revendication (8) **caractérisé en ce que** le non-tissé (10) possède un taux d'élargissement de 1,7% jusqu'à 5,6%, préférentiellement de 2% à 3% et que les perforations ont un rapport des axes MD/CD compris entre 1 et 1,8.
